(19) Europäisches Patentamt
European Patent Office
Office européen des brevets

(11) Veröffentlichungsnummer: **0 051 232**
**B1**

# (12) EUROPÄISCHE PATENTSCHRIFT

(45) Veröffentlichungstag der Patentschrift: **19.09.84**

(51) Int. Cl.³: **C 11 D 3/20**, C 11 D 3/06, A 61 K 7/50

(21) Anmeldenummer: **81108870.7**

(22) Anmeldetag: **24.10.81**

(54) **Handreinigungsmittel.**

(30) Priorität: **30.10.80 DE 3040804**

(43) Veröffentlichungstag der Anmeldung: **12.05.82 Patentblatt 82/19**

(45) Bekanntmachung des Hinweises auf die Patenterteilung: **19.09.84 Patentblatt 84/38**

(84) Benannte Vertragsstaaten: **AT BE CH DE FR GB IT LI NL SE**

(56) Entgegenhaltungen:
**CA - A - 874 942**
**CH - A - 459 430**
**FR - A - 1 552 240**

**H. Janistyn, Handbuch der Kosmetika und Riechstoffe, Band 1, (1978), Hüthig Verlag Heidelberg, Seiten 236-239**
**Beilsteins Handbuch der organischen Chemie, 4. Auflage, drittes Ergänzungswerk, Band II, 1 (1960), Seite 308**
**G.G. HAWLEY, Condensed Chemical Dictionary, (1977), ninth edition, page 289**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Zetzsche, Friedbert, Elbruchstrasse 6, D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Gegenstand der Erfindung sind Handreinigungsmittel, die zur Entfernung von Öl, Fett, Pigmentschmutz, Bitumen, frischer Farbe sowie Druckfarben geeignet sind, in Form einer homogenen, wäßrigen, Tenside, Phosphate und andere Zusatzstoffe enthaltenden Lösung.

In der Praxis läßt sich nicht vermeiden, daß bei einer Reihe von Arbeiten eine Verschmutzung der Hände mit Ölen, Fetten, Pigmenten, Bitumen, frischen Farben sowie Druckfarben erfolgt.

Es ist bereits bekannt, für die Entfernung der oben angeführten Verschmutzungen Handreinigungsmittel zu verwenden. Die Ergebnisse sind nicht immer voll zufriedenstellend, da die Mittel außer der Entfernung des Schmutzes unter anderem gute Hautverträglichkeit aufweisen sowie geruchsarm sein sollen. Auch ist es erwünscht, daß keine brennbaren Flüssigkeiten als Bestandteil eingesetzt werden und keine gesundheitliche Gefahr durch Lösungsmitteldämpfe erfolgt. Auch sind der Anwendungstemperatur der Mittel durch die Empfindlichkeit der Haut Grenzen gesetzt.

Es wurde nun gefunden, daß man unter Vermeidung der oben angeführten Schwierigkeiten eine Entfernung des Schmutzes, insbesondere von den Händen, mit Hilfe einer homogenen, wäßrigen, Tenside, Phosphate und andere Zusatzstoffe enthaltenden Lösung erzielt, wenn man sich der nachstehend beschriebenen Handreinigungsmittel bedient. Diese sind dadurch gekennzeichnet, daß sie

a) 7—67 Gewichtsteile Tenside
b) 1— 8 Gewichtsteile wasserlösliche Phosphate
c) 4—43 Gewichtsteile Essigsäure-(n-butoxy-2-äthoxy-äthyl)-ester
d) 0—14 Gewichtsteile Lösungsvermittler
e) 28—88 Gewichtsteile Wasser

enthalten. Die unter c) angeführte Verbindung kann auch als »n-Butoxy-2-äthoxy-äthyl-acetat« gemäß der Formel

$$CH_3-COO[(CH_2)_2-O-(CH_2)_2-O-C_4H_9]$$

bezeichnet werden. Zur Vereinfachung wird auch die Abkürzung EBE benutzt.

Es wurde weiterhin gefunden, daß zweckmäßigerweise als Tenside wasserlösliche Fettsäureseifen und nichtionogene Tenside verwendet werden können.

Als Fettsäureseifen kommen die Natriumsalze, vorzugsweise jedoch die Kaliumsalze von Fettsäuren mit 14 bis 20, vorzugsweise 16 bis 18, C-Atomen in Betracht.

Als geeignete nichtionogene Tenside sind zu nennen die Anlagerungsprodukte von Ethylen- oder Propylenoxid an Polypropylenglykol beziehungsweise Polyethylenglykol sowie Anlagerungsprodukte von Ethylen- und/oder Propylenoxid an Mono- oder Polyalkohole, Mono- oder Polyamine, Fettsäuren, Amide und Alkylphenole mit einem Alkylrest von vorzugsweise 8 bis 20, insbesondere 12 bis 18 C-Atomen sowie Fettsäurealkanolamide und Fettsäurepolyalkanolamide.

Daneben können jedoch auch amphotere Tenside, wie beispielsweise Kokosdimethylammoniumbetain oder Alkylaminethosulfate, Verwendung finden. Schließlich kommen auch anionenaktive Tenside, wie Alkylbenzolsulfonat, Alkylsulfate, Alkylethosulfate und Alkylsulfonate (mit jeweils 8 bis 18 Kohlenstoffatomen im Alkylrest) in Betracht.

Es hat sich bei Verwendung der amphoteren Tenside als vorteilhaft erwiesen, wenn diese im Verhältnis zum Gesamttensidgehalt in einer Menge von 1 : 3 bis 1 : 10 Teilen eingesetzt werden.

Schließlich werden wasserlösliche Phosphate in Form der Natrium-, Kalium- und Ammoniumphosphate, insbesondere polymere Phosphate wie Alkalidiphosphat und Alkalitriphosphat in Form der Natrium- vorzugsweise Kaliumsalze verwendet.

Weiterhin können die oben beschriebenen Mittel noch 0 bis 14 Gewichtsteile Lösungsvermittler enthalten. Als Lösungsvermittler kommen insbesondere Natriumisopropylbenzolsulfonat und/oder Natriumethylenglykolmonobutylethersulfat in Betracht.

In H. Janistyn, Handbuch der Kosmetika und Riechstoffe, Band I, (1978), Seiten 236—239, sind mehr oder minder summarisch die Eigenschaften der verschiedenen Diethylenglykolderivate angegeben, ohne daß Hinweise auf die Verwendung von Handreinigungsmittel erwähnt werden. Aus CA-A-874 942 waren bereits wäßrige Handreinigungsmittel bekannt, die Tenside und Phosphate neben anderen Verbindungen enthalten. Die Zusammensetzung der beanspruchten Mittel wird hierdurch nicht nahegelegt. Es war nicht zu erwarten, daß mit der speziellen Kombination der beanspruchten Handreinigungsmittel besonders gute Ergebnisse erhalten werden.

Die Verbindung Essigsäure-(n-butoxy-2-äthoxy-äthyl)-ester ist aus Beilsteins Handbuch der organischen Chemie, 4. Auflage, drittes Ergänzungswerk, Band II, 1 (1960) Seite 308, als gutes Lösungsmittel bekannt.

Die oben beschriebenen neuen Mittel zur Entfernung der angeführten Verschmutzungen können bei Zimmertemperatur oder auch erhöhten Temperaturen angewendet werden. Sie lassen sich leicht mit kaltem oder warmem Wasser abspülen und zeigen eine gute Hautverträglichkeit. Weiterhin haben sie den Vorteil, daß sie geruchsarm und weder entzündlich noch brennbar sind.

Mit den erfindungsgemäßen Mitteln können auch starre Oberflächen von entsprechenden Verunreinigungen gesäubert werden.

Der Anmeldungsgegenstand wird durch die nachstehenden Beispiele erläutert.

Beispiel 1

63,0 Gewichtsteile Octylalkohol + 4 Mol Ethylenoxid
4,0 Gewichtsteile $C_{12}$-Alkylbenzolsulfonat
1,0 Gewichtsteile Kaliumtriphosphat
4,0 Gewichtsteile Essigsäure-(n-butoxy-2-äthoxy-äthyl)-ester
28,0 Gewichtsteile Wasser

Beispiel 2

2,4 Gewichtsteile Oleyl-Cetylalkohol + 4 Mol Ethylenoxid
5,4 Gewichtsteile Fettsäurediethanolamid
2,1 Gewichtsteile Kokosdimethylammoniumbetain
4,0 Gewichtsteile Kalium-Seife (Kettenlänge $C_{16}$—$C_{18}$)
9,1 Gewichtsteile Natrium-isopropylbenzolsulfonat
20,0 Gewichtsteile EBE
3,2 Gewichtsteile Natriumtriphosphat
53,8 Gewichtsteile Wasser

Beispiel 3

14,0 Gewichtsteile Kokosfettsäurediethanolamid
43,0 Gewichtsteile EBE
13,6 Gewichtsteile Natrium-isopropylbenzolsulfonat
1,0 Gewichtsteile Natriumtriphosphat
28,4 Gewichtsteile Wasser

Beispiel 4

15,0 Gewichtsteile Nonylphenol + 6 Mol EO
10,0 Gewichtsteile EBE
10,0 Gewichtsteile Natrium-ethylenglykolmonobutyl-ethersulfat
5,0 Gewichtsteile Kaliumdiphosphat
60,0 Gewichtsteile Wasser

Beispiel 5

2,4 Gewichtsteile Kalium-Seife (Kettenlänge $C_{16}$—$C_{18}$)
4,6 Gewichtsteile Nonylphenol + 8 Mol Ethylenoxid
4,0 Gewichtsteile EBE
2,0 Gewichtsteile Kaliumdiphosphat
87,0 Gewichtsteile Wasser

Beispiel 6

9,2 Gewichtsteile Natriumlaurylethosulfat
2,0 Gewichtsteile Natriumalkylaminethosulfat
8,0 Gewichtsteile Kaliumtriphosphat
7, 0 Gewichtsteile EBE
3,5 Gewichtsteile Natriumisopropylbenzolsulfonat
88,0 Gewichtsteile Wasser

Die in den obigen Beispielen 1 bis 6 beschriebenen Mittel sind vorzüglich geeignet, die Hände von Öl, Fett, Bitumen, frischer Farbe usw. zu reinigen und finden bei Raumtemperatur oder auch erhöhten Temperaturen Anwendung. Die Mittel lassen sich leicht wieder durch Wasser abspülen.

## Patentansprüche

1. Handreinigungsmittel in Form einer homogenen, wäßrigen, Tenside und Phosphate enthaltenden Lösung, dadurch gekennzeichnet, daß sie

a) 7 bis 67 Gewichtsteile Tenside
b) 1 bis 8 Gewichtsteile wasserlösliche Phosphate
c) 4 bis 43 Gewichtsteile Essigsäure-(n-butoxy-2-äthoxy-äthyl)-ester
d) 0 bis 14 Gewichtsteile Lösungsvermittler
e) 28 bis 88 Gewichtsteile Wasser

enthalten.

2. Mittel nach Anspruch 1, dadurch gekennzeichnet, daß sie als Tensid wasserlösliche Fettsäureseifen und nichtionogene Tenside enthalten.

3. Mittel nach Anspruch 1 und 2, dadurch gekennzeichnet, daß sie Tensidkombinationen mit einem Anteil an amphoteren Tensiden im Verhältnis zum Gesamttensidgehalt von 1 : 3 bis 1 : 10 enthalten.

4. Mittel nach Anspruch 1 bis 3, dadurch gekennzeichnet, daß sie als wasserlösliche Phosphate Natrium- oder Kaliumtriphosphat enthalten.

5. Mittel nach Anspruch 1 bis 4, dadurch gekennzeichnet, daß sie als Lösungsvermittler Natrium-isopropylbenzolsulfonat und/oder Natriumethylenglykolmonobutylethersulfat enthalten.

## Claims

1. Hand cleaning preparations in the form of a homogeneous aqueous solution containing surfactants and phosphates, characterized in that they contain

a) from 7 to 67 parts by weight of surfactants,
b) from 1 to 8 parts by weight of water-soluble phosphates,
c) from 4 to 43 parts by weight of acetic acid(n-butoxy-2-ethoxyethyl)-ester,
d) from 0 to 14 parts by weight of solution promoter,
e) from 28 to 88 parts by weight of water.

2. Preparations as claimed in claim 1, characterized in that they contain water-soluble fatty acid soaps and non-ionic surfactants as the surfactant.

3. Preparations as claimed in claims 1 and 2, characterized in that they contain surfactant combinations with a proportion of amphoteric surfactants in relation to the total surfactant content of from 1 : 3 to 1 : 10.

4. Preparations as claimed in claims 1 to 3, characterized in that they contain sodium or potassium triphosphate as water-soluble phosphates.

5. Preparations as claimed in claims 1 to 4, characterized in that they contain sodium isopropyl benzene sulfonate and/or sodium ethylene glycol monobutyl ether sulfate as solution promoters.

## Revendications

1. Produits pour le nettoyage des mains à l'état de solutions aqueuses homogènes contenant des agents tensioactifs et des phosphates, caractérisés en ce qu'ils contiennent:

a) de 7 à 67 parties en poids d'agents tensioactifs,
b) de 1 à 8 parties en poids de phosphates hydrosolubles,
c) de 4 à 43 parties en poids d'acétate de n-butoxy-2-éthoxyéthyle,
d) de 0 à 14 parties en poids d'agents solubilisants,
e) de 28 à 88 parties en poids d'eau.

2. Produits selon la revendication 1, caractérisé en ce qu'ils contiennent en tant qu'agent tensioactifs des savons d'acides gras et agents tensioactifs non ioniques solubles dans l'eau.

3. Produits selon les revendications 1 et 2, caractérisés en ce qu'ils contiennent une combinaison d'agents tensioactifs avec des agents tensioactifs amphotères à un rapport de 1 : 3 à 1 : 10 avec la teneur totale en agents tensioactifs.

4. Produits selon les revendications 1 à 3, caractérisé en ce qu'ils contiennent en tant que phosphate soluble dans l'eau du triphosphate de sodium ou du triphosphate de potassium.

5. Produits selon la revendications 1 à 4, caractérisés en ce qu'ils contiennent en tant qu'agent solubilisant de l'isopropylbenzènesulfonate de sodium et/ou le sel de sodium du sulfate de l'éther monobutylique de l'éthylèneglycol.